(19) Europäisches Patentamt
European Patent Office
Office européen des brevets

(11) Publication number: 0 171 787
A1

(12) # EUROPEAN PATENT APPLICATION

(21) Application number: 85110125.3

(22) Date of filing: 13.08.85

(51) Int. Cl.[4]: C 07 D 203/02
C 07 D 203/08
//A61K31/395

(30) Priority: 13.08.84 JP 167929/84
13.08.84 JP 167930/84

(43) Date of publication of application:
19.02.86 Bulletin 86/8

(84) Designated Contracting States:
CH DE FR GB IT LI

(71) Applicant: RESEARCH ASSOCIATION FOR UTILIZATION OF LIGHT OIL
4-11, Shiba 3-chome Minato-ku
Tokyo(JP)

(72) Inventor: Kitagawa, Sadao
544-125, Wakaguri Ami-Machi
Inashiki-Gun Ibaraki-Ken(JP)

(72) Inventor: Yokoi, Takashi
c/o Shin Wakaguri Ryo 1315, Wakaguri
Ami-Machi Inashiki-Gun Ibaraki-Ken(JP)

(72) Inventor: Kaitoh, Mitsumasa
6-14, 4668, Ami Ami-Machi
Inashiki-Gun Ibaraki-ken(JP)

(74) Representative: Hassler, Werner, Dr.
Postfach 17 04 Asenberg 62
D-5880 Lüdenscheid(DE)

(54) **Process for production of aziridine-2-carboxylic acid amide.**

(57) A novel process for producing aziridine-2-carboxylic acid amide comprises reacting a 2,3-dichloropropionic acid ester or amide, a 2-chloroacrylic ester or amide, or mixtures thereof with ammonia, at a temperature not lower than 0°C. The process is preferably carried out in the presence of a tertiary amine or a quaternary ammonium salt. The resulting aziridine-2-carboxylic acid amide is readily purified or isolated by treating the reaction liquid with a basic anion exchange resin.

EP 0 171 787 A1

# PROCESS FOR PRODUCTION OF AZIRIDINE-2-CARBOXYLIC ACID AMIDE

## BACKGROUND OF THE INVENTION

### Field of the Art

This invention relates to a process for producing aziridine-2-carboxylic acid amide. More particularly, this invention relates to a process for producing aziridine-2-carboxylic acid amide by the reaction of either a 2,3-dichloropropionic acid ester or amide with ammonia, or a 2-chloroacrylic acid ester or amide with ammonia.

Aziridine-2-carboxylic acid amide itself or a derivative thereof has physiological activities such as antitumorigenic activity. Also, because of its high reactivity, it is useful as an intermediate for preparation of medical drugs, agricultural chemicals, α-amino acids, etc., and as a starting material for functional resins and the like. However, an industrially advantageous process for production of the aziridine-2-carboxylic acid amide has not been established as far as we are aware although there has been an urgent need for such an industrially feasible process.

### Description of the Prior Art

Conventional methods for synthesizing aziridine-2-carboxylic acid amide can be generally classified into the following three groups.

(1) A method comprising reaction of a 2,3-dibromopropionate ester or 2-bromoacrylic acid amide with liquid ammonia (at -26°C) [Switzerland Patent No.362,078 (1957); Helv. Chim. Acta, 49, 359 (1966)].

(2) A method comprising reaction of an aziridine-2-carboxylate ester with liquid ammonia or alcoholic ammonia [Chem. Ber. 93, 1632 (1960); West Germany Patent Laid-Open Specification No.2,748,015 (1976)].

(3) A method comprising partial hydrolysis of aziridine-2-nitrile [East Germany Patent No.129,551 (1977); J. Prakt. Chem. 321, 712 (1979)].

These methods, however, have the following problems according to the best of our knowledge. There is no description as to the yield in the method (1), and, moreover, this method is not industrially advantageous with respect to cost because a bromine compound is used as a starting material. In the case where the corresponding chlorine compound is used instead of the bromine compound in this method, almost no aziridine-2-carboxylic acid amide was obtained (refer to Comparative Example 1 set forth hereinafter). In addition, aziridine-2-carboxylic acid amide is isolated in this method by extracting an aziridine-2-carboxylic acid ester from a reaction liquid, which step is followed by crystallization from the resulting residue. It is considered that the rate of recovery of highly pure aziridine-2-carboxylic acid amide obtained by crystallization will be low although there is no description as to yield. This is because it is difficult to completely separate an ammonium halide by-product from aziridine-2-carboxylic acid amide by crystallization (refer to Comparative Example 2 presented hereinafter).

Also, methods (2) and (3) cannot be said to be industrially feasible because the aziridine compounds to be used as starting materials therein are not easily obtained, although the yields themselves according to these methods are as high as about 80% and about 95%, respectively.

## SUMMARY OF THE INVENTION

We have conducted intensive research with the aim of developing an industrially advantageous process for producing aziridine-2-carboxylic acid amide, and have unexpectedly succeeded in obtaining aziridine-2-carboxylic acid amide in a high yield by using a 2,3-dichloropropionic acid ester or amide, or, alternatively, a 2-chloroacrylic acid ester or amide as a starting material and by reacting the material with ammonia at 0°C to room temperature or under heating. Heretofore the reaction

under such conditions have not been carried out because of the problems with respect to stability of the intermediate and the product. It has also been found that aziridine-2-carboxylic acid amide can be separated in a surprisingly simple manner by using a basic anion-exchange resin for the treatment of the reaction liquid after termination of the reaction.

Thus, the process for producing aziridine-2-carboxylic acid amide according to the present invention in its broadest aspect comprises reacting an ester or amide of 2,3-dichloropropionic acid of the following formula (I), an ester or amide of 2-chloroacrylic acid of the formula (II), or a mixture thereof with ammonia at a temperature not lower than 0°C.

Cl
|
$Cl-CH_2-CH-CO-X$ ........... (I)

Cl
|
$CH_2=C-CO-X$ .......... (II)

wherein, X denotes $NH_2$ or OR wherein R denotes a hydrocarbon residue containing 1 to 10 carbon atoms.

The aziridine-2-carboxylic acid thus produced, which is still crude can be purified by treating the resulting reaction liquid containing aziridine-2-carboxylic acid amide with a basic anion exchange resin to isolate the aziridine-2-carboxylic acid amide from the reaction liquid.

In accordance with the present invention, aziridine-2-carboxylic acid amide can be obtained in a high yield. In addition, the present invention is advantageous with respect to starting materials. More specifically, the 2,3-dichloropropionic acid ester and amide to be used as a starting material can be readily obtained by chlorine-addition reaction of an acrylic ester and acrylic

amide, respectively. Also the 2-chloroacrylic ester and amide used as a starting material can be obtained by the reaction of the above mentioned chlorine-adducts with an alkali and the like. Moreover, purification of the compound is simple in that the reaction liquid containing the resulting aziridine-2-carboxylic acid amide thus produced is, after the ammonium halide and the like contained therein having been precipitated by cooling, concentration or the like are removed or without such pretreatment, contacted with a basic anion exchange resin and concentration of the solvent used and recovered is carried out. Thus, aziridine-2-carboxylic acid amide having high purity can be obtained.

Accordingly, the present invention provides an advantageous process for industrially producing aziridine-2-carboxylic acid amide, which is largely improved with respect to starting materials and costs, on the basis of the above mentioned reaction conditions.

## DETAILED DESCRIPTION OF THE INVENTION

### Synthesis of the compound

#### Starting material (I)

One of the starting materials, a 2,3-dichloropropionic acid ester or amide or a 2-chloroacrylic ester or amide, is represented by the following formula (I) or (II):

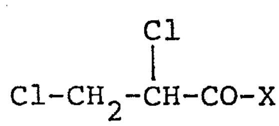
.......... (I)

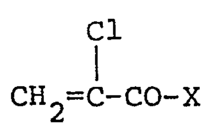
..........(II)

wherein, X stands for OR wherein, R is a hydrocarbon residue containing 1 to 10, preferably 1 to 8 carbon atoms, or $NH_2$.

Thus, the compounds (I) and (II) are preferably an ester having methyl, ethyl, n-propyl, i-propyl, n-butyl,

i-butyl, sec.-butyl, tert.-butyl, n-pentyl, n-hexyl, n-octyl, benzl group or the like; or amide. More preferred is an ester having methyl, ethyl, n-propyl, n-butyl or benzyl group; or amide. Expecially preferred is an ester having methyl, ethyl or benzyl group; or amide.

Starting material (II)

The other starting material is ammonia.

The amount of ammonia to be preferably used for each material (I) is summarized in the following table.

| Material (I) | Suitable amount (molar equivalent/ material (I)) |
|---|---|
| 2,3-dichloropropionate ester | 4 - 200 |
| 2,3-dichloropropionic acid amide | 3 - 200 |
| 2-chloroacrylic ester | 3 - 200 |
| 2-chloroacrylic amide | 2 - 200 |

More preferable amounts of the ammonia are in the same range of 10 to 100 equivalent irrespective of the type of the material (I), and the most preferable amount is 20 to 60 equivalent.

The ammonia which has been used for the reaction can be recovered for reuse.

Solvent

The reaction is conducted either in a solvent-free liquid ammonia or in an ammonia solution containing a solvent miscible with liquid ammonia. There is no large difference in reactivity between the two reaction systems although the optimum reaction temperature and reaction pressure for these reaction systems may differ from each other. Therefore, either reaction system can be selected depending on the circumstances.

The solvents which can be used in the invention include water, an aliphatic alcohol, dimethyl ether, tetrahydrofuran, dimethylsulfoxide, dimethylformamide, hexamethylphosphoric acid triamide, etc. and mixtures

thereof. Among these, water and aliphatic alcohols are preferred. The aliphatic alcohols are preferably those containing 1 to 8 carbon atoms such as methanol, ethanol, n-propanol, i-propanol, n-butanol, i-butanol, sec.-butanol, tert.-butanol, n-pentanol, i-pentanol, n-hexanol, 2-ethylhexanol, n-octanol and the like.

Of these solvents, water, methanol, ethanol, n-propanol, i-propanol, n-butanol, i-butanol, sec.-butanol, tert.-butanol, etc. are preferred. Especially preferred are water, methanol, ethanol, n-propanol, and i-propanol.

Temperature, pressure and duration of reaction

Reaction temperature and time are important factors for the yield of aziridine-2-carboxylic acid amide in the present invention and are ordinarily in the ranges of 0 to 200°C and 0.05 to 48 hours, respectively. Moreover, the combination of reaction temperature and reaction time is also important and varies depending on the kind and concentration of the starting material (I), the kind of solvent, the concentration of ammonia, and other factors. It will be necessary to determine the combination with consideration of the above mentioned factors and particularly the reactivity of the starting material (I) with ammonia as well as decomposition and successive reaction of the resulting aziridine-2-carboxylic acid amide.

More specifically, when the reaction is conducted at a high temperature, for example, of 100°C or more, reaction time is preferably as short as, for example, 4 hours or less, particularly 0.1 to 2 hours. At a relatively low temperature such as 60°C or lower, reaction time is preferably 0.5 hour or longer, especially 1 to 30 hours. Examples of such combinations of the reaction temperature with the reaction time are further shown below.

In the case where a 2,3-dichloropropionic acid ester or amide is to be reacted with 40 equivalent amount of

ammonia for 4 hours, the reaction temperature is ordinarily 0 to 60°C, preferably 10 to 50°C, more preferably 20 to 40°C when the solvent is liquid ammonia itself; whereas, the temperature is ordinarily 10 to 120°C, preferably 40 to 100°C, and more preferably 60 to 80°C when the solvent is a mixture of ammonia and i-propanol in a weight ratio of 1:1.

The reaction proceeds at an autogenous pressure.

Additives

In some cases, increase in yield is observed by adding a tertiary amine, a quaternary ammonium salt or mixtures thereof to the present reaction system.

Examples of tertiary amines which can be used are trimethylamine, triethylamine, tri(n-butyl)amine, tri(2-ethylhexyl)amine, tri(n-octyl)amine, N,N,N',N'-tetramethyl-1,2-diaminoethane, N,N,N',N'-tetramethyl-1,3-diaminopropane, N-methylpiperidine, N-ethylpiperidine, N-methylpyrrolidine, N-ethylpyrrolidine, N,N-dimethylaniline, N,N-diethylaniline, and N,N-dimethylbenzylamine. Among these triethylamine, tri(n-butyl)amine, N-methylpiperidine, and N-methylpyrrolidine are preferred.

Examples of quaternary ammonium salts which can be used are tetramethylammonium hydroxide, trimethylbenzylammonium hydroxide, triethylbenzylammonium hydroxide, tri(n-butyl)benzylammonium hydroxide, tetra(n-butyl) ammonium hydroxide, tri(n-octyl)methylammonium hydroxide, N-(n-lauryl)pyridinium hydroxide, tetramethylammonium hydrogensulfate, trimethylbenzylammonium hydrogensulfate, triethylbenzylammonium hydrogensulfate, tri(n-butyl) benzylammonium hydrogensulfate, tetra(n-butyl)ammonium hydrogensulfate, tri(n-octyl)methylammonium hydrogensulfate, N-(n-lauryl)pyridinium hydrogensulfate, tetramethylammonium chloride, trimethylbenzylammonium chloride, triethylbenzylammonium chloride, tri(n-butyl)benzylammonium chloride, tetra(n-butyl)ammonium chloride, tri(n-octyl) methylammonium chloride, N-(n-lauryl)pyridinium chloride, tetra(n-butyl)ammonium bromide, and tetra(n-butyl)ammonium

iodide. Among these tetramethylammonium hydroxide, trimethylbenzylammonium hydroxide, tetra(n-butyl) ammonium hydroxide, tri(n-octyl)ammonium hydroxide, and tri(n-octyl)methylammonium hydroxide are preferred.

The amount of the additive to be used is the catalytic amount or more.

Reaction operation

For example, a pressure-proof reaction vessel is charged with either a 2,3-dichloropropionic acid ester or amide, or a 2-chloroacrylic ester or amide, a solvent and then with ammonia. Reaction is then caused to proceed by stirring and heating the reaction mixture. After termination of the reaction, the process product after ammonia is purged off is subjected to concentration whereby a solid product is obtained which comprises aziridine-2-carboxylic acid amide. The aziridine-2-carboxylic amide which is crude may be subjected to purification operation including ion-exchange operation or column chromatography thereby to obtain a purified preparation of aziridine-2-carboxylic amide in white crystals.

Purification of the compound

The preferred purification process comprises treating the crude aziridine-2-carboxylic amide with a basic anion-exchange resin.

The reaction liquid obtained from the synthesis step is thus contacted with basic anion exchange resin. Alternatively, the ammonium halide and the like precipitated by cooling, concentration, etc. in the reaction liquid are removed therefrom, and then the reaction liquid is contacted with the resin. Thereafter, the reaction liquid is simply concentrated by removing the solvent to give aziridine-2-carboxylic acid amide having high purity.

In the case where it is not necessary to isolate the aziridine-2-carboxylic acid amide as crystals, the above-mentioned reaction liquid can of course be applied to various uses without concentration by removing the solvent.

By the use of an anion exchange resin, it is possible to remove in a single operation not only halide ions resulting from the reaction but also anionic by-products such as those produced by conversion of the amide to the corresponding carboxylic acid. It is of course possible to recover the anion exchange resin for reuse.

Incidentally, the ammonia distilled off from the reaction liquid after the reaction can also be recovered and repeatedly reused.

Basic anion exchange resin

The basic anion-exchange resin to be used can be either strongly basic or weakly basic. However, in the case where a strongly basic anion exchange resin is used, it should be of OH type. As a substrate for the ion exchange resin, any type of substrate such as a gel type, porous type or macroporous type can be used. Thus, the basic anion exchange resin is not restricted to specific ones, as long as it is a basic anion exchange resin. Strongly basic anion exchange resins are preferred. It is possible to use mixtures of a strongly basic anion exchange resin and a weakly basic one as well as mixtures of two or more basic anion exchange resins of different types. As necessary, a cation exchange resin can be used in combination with an anion exchange resin. For further detail of basic anion exchange resins, reference is made to, inter alia, Jacob A. Marinsky: ION EXCHANGE Vol. 1, Marcel Dekker, Inc., New York (1966) which is herein incorporated as reference.

The ion exchange resin can be in the form of particles, a membrane and other desired forms as long as they are reasonable. The term "particles" herein means spheres, granules, pellets and the like. The term "membrane" herein means films, sheets and the like composed of an anion exchange resin or a composition containing said resin optionally together with a supporting material.

The amount of the anion exchange resin to be used

is the same for both a strongly basic resin and a weakly basic resin, and is 1.0 equivalent or more, preferably 1.2 equivalent or more based on the total amount of anionic by-products such as halide ions, carboxyl groups and the like present in the reaction liquid containing aziridine-2-carboxylic acid amide. Although any amount mentioned above or more can be used, it is not desirable to use 50 equivalents or more from the view point of economy and efficiency. For example, in the case where a granular basic anion exchange resin having an exchange capacity of 1.3 meq. per ml is to be used for the reaction liquid containing 1 mol of ammonium halide and 0.3 mol of aziridine-2-carboxylic acid together with the aziridine-2-carboxylic acid amide resulting from the reaction, the amount of said resin is 1.0 liter or more, preferably 1.2 liters or more. If the amount of said resin is less than 1.0 liter, leakage of anionic by-products such as halide ions and carboxyl compounds will occur.

Operation procedures

Contacting of the reaction liquid containing aziridine-2-carboxylic acid amide with the ion exchange resin may be carried out either successively by means of a column charged with the granular resins or an apparatus using the resin membranes, or, alternatively, in a batchwise system.

For example, in a column-type contact, the reaction liquid containing the resulting aziridine-2-carboxylic acid amide is passed through a column charged with ion exchange resins, after concentrating the reaction liquid to partially precipitate by-products such as an ammonium halide, or without such pretreatment. As necessary, a solvent may be then passed through the column to wash out the product. The solvent is preferably the same as the one used for the reaction liquid. However, water and lower alcohols may be used even in the case where the solvent used for the reaction liquid is

different therefrom. There is no specific limitation as to concentration of the reaction liquid containing the resulting aziridine-2-carboxylic acid amide nor as to the amount of the solvent, provided that the solution has fluidity.

In the case where a membranous ion exchange resin is to be used, it is possible in the present invention to use any of the conventional methods employing an ion exchange membrane such as diffusion dialysis, pressure dialysis, and electrodialysis. The term "diffusion dialysis" herein means a method which comprises producing a difference between the concentrations of the impurities to be removed on opposite sides of a membrane and thereby removing these impurities. The term "pressure dialysis" herein means a method which comprises producing a pressure difference between the opposite sides of a membrane to remove impurities. The term "electrodialysis" herein means a method which comprises applying an electric potential across a membrane to remove impurities.

Furthermore, a granular ion exchange resin and a membranous ion exchange resin can be used in combination in the present invention.

The temperature for treating with the anion exchange resin is not particularly restricted. However, it will be ordinarily in the range of 0° to 60°C, preferably in the range of 10° to 40°C in consideration of thermal resistance of the ion exchange resin.

By concentrating the reaction liquid thus passed through the anion exchange resin crystals of aziridine-2-carboxylic acid amide of high purity can be readily obtained.

The purity of the aziridine-2-carboxylic acid amide can be further increased by, for example, recrystallization or like procedure.

The ion exchange resin thus used can be regenerated for reuse by conventional regeneration procedures employing

an aqueous solution of sodium hydroxide and the like.

Incidentally, it is preferable to store the isolated crystals of aziridine-2-carboxylic acid amide in a dark cool place because they are thermally unstable.

## Experimental Examples

### Example 1

A 300 ml pressure reaction vessel was charged with 5.0 g (31.8 m mol) of methyl 2,3-dichloropropionate and 22 g of water. After the vessel was sealed, 22 g (1.29 mol) of ammonia was added thereto, and then reaction was carried out at 60°C for 8 hours. During this reaction, the pressure within the vessel was 7.5 kg/cm$^2$. After the reaction, the ammonia was purged off, and the resulting reaction liquid was subjected to a liquid chromatography analysis. As a result, the yield of aziridine-2-carboxylic acid amide product was found to be 91.6%.

The aqueous solution of the aziridine-2-carboxylic acid amide thus synthesized was passed through a column charged with 70 ml of a strongly basic anion exchange resin (Diaion SA-10A (OH type)), and then 70 ml of distilled water was passed through the column. This operation was carried out at room temperature. Water was distilled off under reduced pressure from the resulting aqueous solution to obtain 2.63 g of crystals of aziridine-2-carboxylic acid amide. The purity thereof was 92.0% (recovery: 96.6%).

### Examples 2 through 10

In Examples 2 through 10, the procedure in Example 1 was carried out by using 5.0 g (31.8 m mol) of methyl 2,3-dichloropropionate as a starting material and varying the conditons.

The results of Examples 1 through 10 are summarized in Table 1.

Table 1

| Example | Ammonia g(mol) | Solvent (g) | Temperature (°C) | Pressure ($kg/cm^2$) | Period of time (hr) | Yield (%) |
|---|---|---|---|---|---|---|
| 1 | 22 (1.29) | $H_2O$ (22) | 60 | 7.5 | 8 | 91.6 |
| 2 | 22 (1.29) | $H_2O$ (22) | 80 | 12.0 | 2 | 82.1 |
| 3 | 11 (0.65) | $H_2O$ (11) | 80 | 8.0 | 2 | 76.6 |
| 4 | 11 (0.65) | $H_2O$ (33) | 100 | 6.0 | 2 | 74.6 |
| 5 | 22 (1.29) | iPrOH (22) | 80 | 21.0 | 2 | 82.0 |
| 6 | 22 (1.29) | iPrOH (22) | 100 | 30.0 | 0.5 | 76.7 |
| 7 | 22 (1.29) | iPrOH (22) | 60 | 15.0 | 4 | 68.3 |
| 8 | 11 (0.65) | iPrOH (99) | 100 | 4.0 | 4 | 67.3 |
| 9 | 22 (1.29) | MeOH (22) | 80 | 16.0 | 2 | 81.3 |
| 10 | 22 (1.29) | none | 30 | 10.0 | 4 | 66.4 |

Example 11

A 50 ml pressure reaction vessel was charged with 3.2 g (31.7 mmol) of triethylamine and 22 g of isopropanol. 5.0 g (31.8 mmol) of methyl 2,3-dichloropropionate over 15 minutes was then added dropwise while the temperature was maintained at 10 to 20°C. After stirring at 20°C for 15 minutes, the reaction mixture was moved to a 300 ml pressure reaction tube. After sealing the tube, 22 g (1.29 mol) of ammonia was placed therein, and reaction was conducted at 60°C for 4 hours. During this reaction, the pressure within the tube was 15.0 $kg/cm^2$. As a result of analysis by liquid chromatography, the yield of the aziridine-2-carboxylic acid amide thus produced was 73.8%. By the addition of triethylamine, the yield of aziridine-2-carboxylic acid amide was increased (refer to Example 7 in Table 1).

Example 12

With a procedure similar to that in Example 11 and by using 5.0 g (31.8 mmol) of methyl 2,3-dichloropropionate, 22 g of isopropanol, 7.5 g (18.0 mmol) of 40% methanolic trimethylbenzylammonium hydroxide solution and 22 g (1.29 mol) of ammonia, reaction was carried out at 80°C for 2 hours (pressure: 19.0 $kg/cm^2$). The yeild of aziridine-2-carboxylic acid amide thus produced was 89.4% (refer to Example 5 in Table 1).

Example 13

Into a 300 ml pressure reaction vessel were charged 4.5g(31.7 mmol) of 2,3-dichloropropionic acid amide and 22 g of water. After the vessel was sealed, 22 g (1.29 mol) of ammonia was added thereto, and then reaction was carried out at 60°C for 8 hours. During this reaction, the pressure within the vessel was 7.5 $kg/cm^2$. After the reaction, the ammonia was purged off, and the resulting reaction liquid was analyzed by liquid chromatography. As a result, the yield of the aziridine-2-carboxylic acid amide product was found to be 90.8%.

Example 14

By using 10.0 g (54.1 mmol) of isopropyl 2,3-dichloropropionate, 37 g of isopropanol and 37 g (2.18 mol) of ammonia and with a procedure similar to that of Example 1, reaction was carried out at 80°C for 2 hours (pressure: 22.0 $kg/cm^2$). The yield of the aziridine-2-carboxylic acid amide thus produced was 51.0%.

Example 15

By using 3.8 g (31.5 mmol) of methyl 2-chloroacrylate ester, 22 g of isopropanol and 22 g (1.29 mol) of ammonia and with a procedure similar to that of Example 1, reaction was carried out at 80°C for 2 hours (pressure: 21.0 $kg/cm^2$). The yield of the aziridine-2-carboxylic acid amide thus produced was 83.3%.

Example 16

By using 3.4 g (32.2 mmol) of 2-chloroacrylic amide, 22 g of water and 22 g (1.29 mol) of ammonia and with a procedure similar to that of Example 1, reaction was carried out at 60°C for 4 hours (pressure: 7.5 $kg/cm^2$). The yield of the aziridine-2-carboxylic acid amide thus produced was 88.8%.

Comparative Example 1

Into a 200 ml glass reaction vessel equipped with a dry ice-cooling tube was placed 120 ml of liquid ammonia, while the vessel was cooled in a dry ice-acetone bath, and 250 mg of N-phenyl-2-naphthylamine was added. Then 4.7 g (29.9 mmol) of methy 2,3-dichloropropionate was added dropwise over 30 minutes. The dry ice-acetone bath was then removed, and reaction was conducted under refluxing of the liquid ammonia (at -26°C) for 3 hours. After the reaction, the cooling tube was removed from the vessel, and the liquid ammonia was distilled off. Then 100 ml of isopropanol was added to the resulting reaction liquid. After white precipitates of ammonium chloride were filtered out, the resulting solution was analyzed by liquid chromatography. As a result, the yield of the aziridine-2-carboxylic acid amide thus produced was found to be only 1.7%.

Example 17

An isopropanol solution of the aziridine-2-carboxylic acid amide synthesized in Example 5 was passed through the column which had been charged with 15 ml of strongly basic anion exchange resin (Diaion SA-10A (OH type)) and preliminarily treated with isopropanol, and then 50 ml of isopropanol was further passed therethrough. This operation was carried out at room temperature. The isopropanol was distilled off from the resulting solution under reduced pressure to produce 2.22 g of crystals of aziridine-2-carboxylic acid amide. The purity of the aziridine-2-carboxylic acid amide was 94.7 % (recovery: 93.6 %).

Example 18

An isopropanol solution of the aziridine-2-carboxylic acid amide synthesized in Example 5 was passed through the column which had been charged with 90 ml of weakly basic anion exchange resin (Diaion WA-10A) and preliminarily treated with isopropanol, and then 100 ml of isopropanol was further passed therethrough. This operation was carried out at room temperature. The isopropanol was distilled off from the resulting solution under reduced pressure to produce 2.32 g of crystals of aziridine-2-carboxylic acid amide. The purity of the aziridine-2-carboxylic acid amide was 92.6 % (recovery: 95.6%).

Comparative Example 2

The isopropanol solution obtained in Example 5 was concentrated to obtain 3.24 g of a pale yellow solid. The purity of the aziridine-2-carboxylic acid amide contained in the solid was only 63.3%.

WHAT IS CLAIMED IS:

1. A process for producing aziridine-2-carboxylic acid amide which comprises reacting a compound selected from the group consisting of an ester or amide of 2,3-dichloropropionic acid represented by the following formula (I), an ester or amide of 2-chloroacrylic acid represented by the following formula (II), and mixtures thereof with ammonia at a temperature not lower than 0°C:

$$\begin{array}{l} \qquad\qquad\quad Cl \\ \qquad\qquad\quad | \\ Cl-CH_2-CH-CO-X \end{array} \qquad \text{........ (I)}$$

$$\begin{array}{l} \qquad\quad Cl \\ \qquad\quad | \\ CH_2=C-CO-X \end{array} \qquad \text{........(II)}$$

wherein, X denotes $NH_2$ or OR wherein R denotes a hydrocarbon residue having 1 to 10 carbon atoms.

2. The process according to Claim 1, wherein the reaction is carried out in a solvent selected from the group consisting of water, an aliphatic alcohol having 1 to 8 carbon atoms and mixtures thereof.

3. The process according to Claim 1 or 2, wherein the reaction is carried out at 0 to 200°C.

4. The process according to Claim 1, 2 or 3, wherein the reaction is carried out in the presence of a compound selected from tertiary amines, quaternary ammonium salts, and mixtures thereof.

5. The process according to Claim 1, 2, 3 or 4, wherein the aziridine-2-carboxylic acid amide is isolated from the reaction liquid by treating the reaction liquid thus produced with a basic anion exchange resin.

European Patent Office

**0171787**

Application number

# EUROPEAN SEARCH REPORT

EP 85110125.3

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| D,A | HELVETICA CHIMICA ACTA, vol. 49, 1966, Basel<br>E. KYBURZ et al. "Synthese und Eigenschaften von Aziridincarbonsäureestern" pages 359-369<br>* Page 365, lines 16-33; page 361, lines 20-23 *<br>-- | 1 | C 07 D 203/02<br>C 07 D 203/08<br>//A 61 K 31/395 |
| D,A | CH - A - 362 078 (HOFFMANN-LA ROCHE)<br>* Example 5 *<br>-- | 1 | |
| A | EP - A1 - 0 030 475 (MITSUI TOATSU CHEMICALS)<br>* Example 8; claim 4 *<br>-- | 1-3 | TECHNICAL FIELDS SEARCHED (Int Cl 4) |
| D,A | DE - A1 - 2 748 015 (INSTITUT ORGANITSCHESKOGO SINTEZA)<br>* Page 5, lines 4-15 *<br>-- | 1-3 | C 07 D 203/00 |
| A | DE - A - 2 200 305 (DOW CHEMICAL CO.)<br>* Claims 1,6; example 2 *<br>---- | 1-3 | |

The present search report has been drawn up for all claims

| Place of search | Date of completion of the search | Examiner |
|---|---|---|
| VIENNA | 30-10-1985 | KÖRBER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82